Europäisches Patentamt

European Patent Office

Office européen des brevets

(19)

(11) Veröffentlichungsnummer: **0 307 655 B1**

## EUROPÄISCHE PATENTSCHRIFT

(12)

(45) Veröffentlichungstag der Patentschrift: **05.08.92**

(51) Int. Cl.⁵: **A61F  2/38**

(21) Anmeldenummer: **88113554.5**

(22) Anmeldetag: **20.08.88**

(54) **Kniegelenk-Endoprothese.**

(30) Priorität: **15.09.87 DE 3730928**

(43) Veröffentlichungstag der Anmeldung:
**22.03.89 Patentblatt  89/12**

(45) Bekanntmachung des Hinweises auf die
Patenterteilung:
**05.08.92 Patentblatt  92/32**

(84) Benannte Vertragsstaaten:
**AT BE CH DE ES FR GB GR IT LI LU NL SE**

(56) Entgegenhaltungen:
**DE-A- 2 805 744**
**DE-A- 2 927 880**
**DE-A- 3 529 894**

(73) Patentinhaber: **S + G IMPLANTS GMBH**
**Grapengiesserstrasse 34**
**W-2400 Lübeck(DE)**

(72) Erfinder: **Grundei, Hans**
**Hamburger Strasse 89**
**W-2400 Lübeck(DE)**
Erfinder: **Thomas, Wolfram, Prof. Dr.**
**Poppenbütteler Landstrasse 12**
**W-2000 Hamburg-Poppenbüttel(DE)**
Erfinder: **Scholz, Jörg, Dr.**
**Oberhaardter Weg 31**
**W-1000 Berlin 33(DE)**

(74) Vertreter: **Eisenführ, Speiser & Partner**
**Martinistrasse 24**
**W-2800 Bremen 1(DE)**

EP 0 307 655 B1

## Beschreibung

Die Erfindung bezieht sich auf eine zu implantierende Kniegelenk-Endoprothese, deren Tibia- und Femurteil kurze Stiele mit einer Länge bis etwa 12 cm aufweisen.

Beim Implantieren der Tibia- und Femurteile einer Kniegelen-Endoprothese kann der Fall eintreten, daß der Stiel nicht zentriert in den Markraum des Schienbeines oder Femurs eindringt, so daß das Stielende durch die exzentrische Lage mit Teilen einen von innen nach außen gerichteten Druck auf die Knochenwandung ausübt, was nach bestimmter Zeit zu einer äußeren Knochenauswölbung führt. Dadurch trifft eine Schwächung des Knochengefüges an dieser Stelle auf, was zu einem Knochenbruch führen kann.

Die Aufgabe der Erfindung besteht darin, den kurzen Stiel von Tibia- und Femurteilen einer zu implantierenden Kniegelenk-Endoprothese so zu führen, daß stärkere einseitige von innen nach außen gerichtete Drücke auf die Knochenwandung vermieden werden.

Diese Aufgabe wird durch die kennzeichnenden Merkmale des Anspruches 1 gelöst.

Vorteilhaft ist es, daß der Stiel am Ende mit einer konischen Ausnehmung zum selbsthemmenden Eingriff des Konus der Sonde versehen ist.Er kann aber auch mit einem Gewindeansatz versehen sein. Dabei kann die Sonde vorteilhaft aus einem Kunststoff oder auch ganz oder auf einer Teillänge aus einem nachgiebigem resorbierbarem Material hergestellt sein.

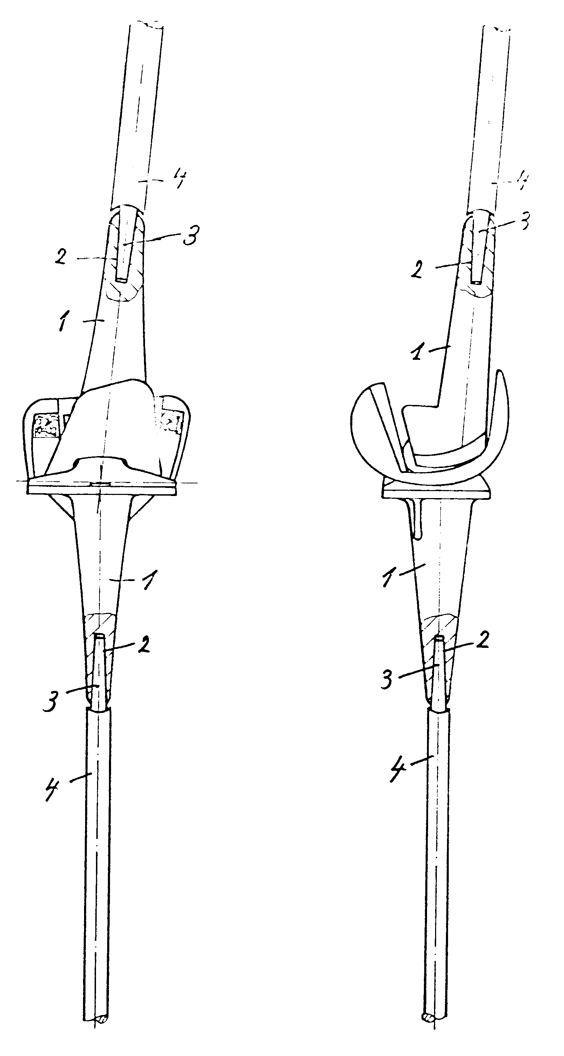

Die Erfindung wird nachstehend anhand der Zeichnung erläutert, in der eine Vorder- und Seitenansicht einer Kniegelenk-Endoprothese mit zum Teil geschnittenen Stielen und mit dem Stiel verbundener,nachgiebiger Sonde dargestellt ist.

Der metallische Stiel 1 des Tibia- und Femurteils einer zu implantierenden Kniegelenk-Endoprothese wird vorteilhaft mit einem äußeren offenzelligen metallischen Belag versehen und ist am Ende mit einer konischen Ausnehmung ausgerüstet, in die das Konusende 3 einer Sonde 4 vorteilhaft selbsthemmend eingreift. Es ist aber auch möglich, die Sonde 4 mit einem Gewindezapfen zu versehen, der in ein Gewinde einer Ausnehmung des Stielendes einschraubbar ist. Die Sonde 4 besteht aus einem nachgiebigem Material, wie z.B. einem geeigneten Kunststoff, und dient als Führung für den zu zentrierenden Stiel 1 beim Einsetzen in den Markkanal des Femurs oder Schienbeins, so daß der Stiel 1 zentriert geführt wird und dadurch eine einseitige Abweichung des Stielendes mit Druck auf die Knochenwandung eindeutig vermieden wird.

Es ist auch möglich, die nachgiebige Sonde ganz oder teilweise aus einem Material herzustellen, welches nach dem Implantieren resorbiert wird.

Solche Materialien sind als solche bekannt.

## Patentansprüche

1. Eine zu implantierende Kniegelenk-Endoprothese, deren Tibia-und Femurteil kurze Stiele (1) mit einer Länge bis zu etwa 12 cm aufweisen, dadurch gekennzeichnet, daß das Ende beider kurzen Stiele (1) zur Führung im Markraum des Knochens durch eine glattwandige Sonde (4) aus nachgiebigem Material verlängert ist.

2. Kniegelenk-Endoprothese nach Anspruch 1, dadurch gekennzeichnet, daß die Stiele (1) am Ende mit einer konischen Ausnehmung (2) zum selbsthemmenden Eingriff eines Konus (3) der Sonde (4) versehen sind.

3. Kniegelenk-Endoprothese nach Anspruch 1 oder 2, dadurch gekennzeichnet, daß die Stiele (1) am Ende mit einer ein Gewinde aufweisenden Ausnehmung (2) versehen sind, in die ein Gewindezapfen der Sonde (4) einschraubbar ist.

4. Kniegelenk-Endoprothese nach einem der Ansprüche 1 bis 3, dadurch gekennzeichnet, daß die Sonde (4) aus einem nachgiebigem Kunststoff besteht.

5. Kniegelenk-Endoprothese nach einem der Ansprüche 1 bis 4, dadurch gekennzeichnet, daß die Sonde (4) mindestens auf einer Teillänge aus einem resorbierbarem Material besteht.

## Claims

1. A kneejoint endoprosthesis for implanting, of which the tibia and femur parts exhibit short stems (1) of a length of up to about 12 cm, characterized in that for guidance into the medullary space in the bone the end of each short stem (1) is prolonged by a smooth-walled probe (4) of resilient material.

2. A kneejoint endoprosthesis as in Claim 1, characterized in that each stem (1) is provided with a conical recess (2) for self-locking engagement of a cone (3) on the probe (4).

3. A kneejoint endoprosthesis as in Claim 1 or 2, characterized in that each stem (1) is provided at the end with a conical recess (2) which exhibits a thread into which a threaded stud on the probe (4) my be screwed.

4. A kneejoint endoprosthesis as in one of the Claims 1 to 3, characterized in that the probe (4) consists of a resilient plastics.

5. A kneejoint endoprosthesis as in one of the Claims 1 to 4, characterized in that the probe (4) over at least part of its length consists of a resorbable material.

**Revendications**

1. Endoprothèse pour l'articulation du genou qui est à implanter et dont la partie de tibia et la partie de fémur présentent de courtes tiges (1) d'une longueur allant jusqu'à environ 12 cm, caractérisée en ce que l'extrémité des deux courtes tiges (1) est, pour le guidage dans la cavité médullaire de l'os, prolongée par une sonde à paroi lisse (4) en une matière flexible.

2. Endoprothèse pour l'articulation du genou suivant la revendication 1, caractérisée en ce que les tiges (1) sont pourvues, à l'extrémité, d'un évidement conique (2) pour la pénétration autobloquante d'un cône (3) de la sonde (4).

3. Endoprothèse pour l'articulation du genou suivant l'une des revendications 1 et 2, caractérisée en ce que les tiges (1) sont pourvues, à l'extrémité, d'un évidement (2) présentant un taraudage dans lequel un tenon fileté de la sonde (4) peut être vissé.

4. Endoprothèse pour l'articulation du genou suivant l'une des revendications 1 à 3, caractérisée en ce que la sonde (4) est constituée d'une matière synthétique flexible.

5. Endoprothèse pour l'articulation du genou suivant l'une des revendications 1 à 4, caractérisée en ce que la sonde (4) est constituée, au moins sur une longueur partielle, d'une matière résorbable.